(19) Europäisches Patentamt
European Patent Office
Office européen des brevets

(11) Veröffentlichungsnummer : **0 049 783**
**B1**

(12) # EUROPÄISCHE PATENTSCHRIFT

(45) Veröffentlichungstag der Patentschrift : **22.10.86**

(51) Int. Cl.[4] : **C 07 J 63/00**, A 61 K 31/12

(21) Anmeldenummer : **81107457.4**

(22) Anmeldetag : **19.09.81**

(54) **D-Homo-kortikoide, ihre Herstellung und Verwendung.**

(30) Priorität : **10.10.80 DE 3038855**

(43) Veröffentlichungstag der Anmeldung : **21.04.82 Patentblatt 82/16**

(45) Bekanntmachung des Hinweises auf die Patenterteilung : **22.10.86 Patentblatt 86/43**

(84) Benannte Vertragsstaaten : **AT BE CH DE FR GB IT LI LU NL SE**

(56) Entgegenhaltungen :
**EP-A- 0 003 341**
**DE-A- 2 349 023**
**FR-A- 2 182 911**
**FR-A- 2 244 474**
**FR-A- 2 283 674**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT Berlin und Bergkamen**
**Müllerstrasse 170/178 Postfach 65 03 11**
**D-1000 Berlin 65 (DE)**

(72) Erfinder : **Annen, Klaus, Dr.**
**Seegefelder Strasse 194**
**D-1000 Berlin 20 (DE)**
Erfinder : **Laurent, Henry, Dr.**
**Glambecker Weg 21**
**D-1000 Berlin 28 (DE)**
Erfinder : **Hofmeister, Helmut, Dr.**
**Weislingenstrasse 4**
**D-1000 Berlin 28 (DE)**
Erfinder : **Wiechert, Rudolf, Prof.**
**Petzower Strasse 8 A**
**D-1000 Berlin 39 (DE)**
Erfinder : **Wendt, Hans, Dr.**
**Ernst-Ring-Strasse 14**
**D-1000 Berlin 38 (DE)**
Erfinder : **Albring, Manfred, Dr.**
**Am Dachsbau 34 a**
**D-1000 Berlin 27 (DE)**

**EP 0 049 783 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft den in den Patentansprüchen gekennzeichneten Gegenstand.

Die D-homo-kortikoide der allgemeinen Formel I gemäß Anspruch 1, können als Substituenten X ein Wasserstoffatom, ein Fluoratom oder ein Chloratom tragen.

Als Substituenten $R_1$ können die D-homo-kortikoide ein Wasserstoffatom oder eine niedere Alkylgruppe tragen. Unter einer niederen Alkylgruppe soll vorzugsweise eine Gruppe mit 1 bis 4 Kohlenstoffatomen verstanden werden, wie zum Beispiel die Methylgruppe, die Äthylgruppe, die Propylgruppe, die Isopropylgruppe oder die Butylgruppe.

Die D-homo-kortikoide der allgemeinen Formel I gemäß Anspruch 1, können als Substituenten Y eine Hydroxygruppe, ein Fluoratom, ein Chloratom oder eine Acyloxygruppe tragen. Unter einer Acyloxygruppe $R_2$ soll eine Gruppe verstanden werden, welche man bei Kortikoiden üblicherweise als Estergruppe in der 21-Position des Moleküls verwendet. Geeignete Acyloxygruppen sind beispielsweise Gruppen mit 1 bis 16 Kohlenstoffatomen, die sich von geradkettigen oder verzweigten, gesättigten oder ungesättigten aliphatischen Mono- oder Dicarbonsäuren ableiten, welche in üblicher Weise, beispielsweise durch Hydroxygruppen, Aminogruppen oder Halogenatome substituiert sein können.

Ferner eignen sich als Acyloxygruppen auch Reste cycloaliphatischer, aromatischer, gemischt aromatisch-aliphatischer oder heterocyclischer Säuren, die ebenfalls in üblicher Weise substituiert sein können. Als geeignete Acyloxygruppen seien beispielsweise genannt:

Die Formyl-, Acetyl-, Propionyl-, Butyryl-, Pentanoyl-, Hexanoyl-, Octanoyl-, Undecanoyl-, Dimethylacetyl-, Trimethylacetyl-, Diäthylacetyl-, tert.-Butylacetyl-, Benzoyl-, Phenacetyl-, Cyclopentylpropionyl-, Hydroxyacetyl-, Monochloracetyl-, Dichloracetyl-, Trichloracetyl-, ferner die Dimethylaminoacetyl-, die Trimethylaminoacetyl-, die Diäthylaminoacetyl-, die Piperidinoacetyl-, die Nicotinoyl-, die ω-Carboxypropionyl- und die ω-Carboxypentanoylgruppe.

Bevorzugte Acyloxygruppe Y sind solche, die 1 bis 8 Kohlenstoffatome besitzen und insbesondere solche, die sich von einer 1 bis 6 Kohlenstoffatome enthaltenden geradkettigen oder verzweigten Alkancarbonsäure ableiten.

D-Homo-kortikoide wie zum Beispiel der 11β,17α-Dihydroxy-3,20-dioxo-D-homo-1,4-pregnadien-21-säure-butylester und das 21-Acetoxy-11β,17α-dihydroxy-D-homo-1,4-pregnadien-3,20-dion sind vorbekannt (siehe z. B. die FR-A-2 182 911, FR-A-2 244 744, die FR-A-2 283 674 und die DE-A-2 349 023). Aus der EP-A-0 003 341 sind auch 17α-Acetale und 17α-Thioacetale von Kortikoiden, wie zum Beispiel das 11β,21-Dihydroxy-17α-methoxymethoxy-1,4-pregnadien-3,20-dion und das 21-Acetoxy-11β-hydroxy-17α-methylthiomethoxy-1,4-pregnadien-3,20-dion vorbekannt.

Die erfindungsgemäßen D-Homo-kortikoide haben gegenüber diesen vorbekannten strukturanalogen Verbindungen den Vorzug, daß sie bei topischer Applikation eine stärkere antiinflammatorische Wirksamkeit entfalten und/oder daß sie eine günstigere Dissoziation zwischen erwünschter topischer Wirksamkeit und unerwünschter systemischer Wirkung besitzen.

Die neuen D-Homo-kortikoide eignen sich in Kombination mit den in der galenischen Pharmazie üblichen Trägermitteln zur lokalen Behandlung von Kontaktdermatitis, Ekzemen der verschiedensten Art, Neurodermatosen, Erythrodermie, Verbrennungen, Pruritis vulvae et ani, Rosacea, Erythematodes cutaneus, Psoriasis, Lichen ruber planus et verrucosus und ähnlichen Hauterkrankungen.

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremen oder Pflaster, überführt. In den so formulierten Arzneimitteln ist die Wirkstoffkonzentration von der Applikationsform abhängig. Bei Lotionen und Salben wird vorzugsweise eine Wirkstoffkonzentration von 0,001 % bis 1 % verwendet.

Darüberhinaus sind die neuen Verbindungen gegebenenfalls in Kombination mit den üblichen Trägermitteln und Hilfsstoffen auch gut zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die neuen Kortikoide auch in Form von Kapseln, Tabletten oder Dragees, die vorzugsweise 10 bis 200 mg Wirkstoff enthalten und oral appliziert werden oder in Form von Suspensionen, die vorzugsweise 100 bis 500 mg Wirkstoff pro Dosiseinheit enthalten und rektal appliziert werden. Auch zur Behandlung allergischer Erkrankungen des Darmtraktes, wie der Kolitis ulcerosa und der Kolitis granulomatosa.

Die neuen D-Homo-kortikoide können nach dem im Patentanspruch 12 gekennzeichneten Verfahren hergestellt werden, welches unter den Bedingungen durchgeführt werden kann, wie sie in EP-A-0 003 541 beschrieben sind.

Die nachfolgenden Ausführungsbeispiele dienen zur Erläuterung des erfindungsgemäßen Verfahrens.

### Beispiel 1

21-Acetoxy-11β-hydroxy-17a-methoxymethoxy-D-homo-1,4-pregnadien-3,20-dion

a) Eine Lösung von 7,32 ml Trifluoressigsäureanhydrid in 61 ml Pyridin wird auf — 20 °C abgekühlt und mit 12,2 g 21-Acetoxy-11β,17a-dihydroxy-D-homo-1,4-pregnadien-3,20-dion versetzt. Man rührt 20 Minuten bei — 15 °C weiter und arbeitet wie üblich auf. Ausbeute 13,9 g 21-Acetoxy-17a-hydroxy-11β-trifluoracetoxy-D-homo-1,4-pregnadien-3,20-dion.

b) 13,2 g 21-Acetoxy-17a-hydroxy-11β-trifluoracetoxy-D-homo-1,4-pregnadien-3,20-dion werden in 97,3 ml wasserfreiem Methylenchlorid und 62,6 ml Formaldehyddimethylacetal gelöst und portionsweise mit einem Gemisch aus 21,0 g Kieselgur W 20 und 10,4 g Phosphorpentoxid versetzt. Man rührt 24 Stunden bei Raumtemperatur nach, saugt ab und eluiert den Rückstand nochmals mit Methylenchlorid, das 3 bis 5 % Triethylamin enthält. Das Rohprodukt wird an 700 g Kieselgel mit einem Methylenchlorid-Aceton-Gradienten (0-15 % Aceton) gereinigt. Ausbeute 7,8 g 21-Acetoxy-17a-methoxymethoxy-11β-trifluoracetoxy-D-homo-1,4-pregnadien-3,20-dion.

c) Man löst 5,8 g 21-Acetoxy-17a-methoxymethoxy-11β-trifluoracetoxy-D-homo-1,4-pregnadien-3,20-dion in 260 ml Methanol und nach Zugabe von 43,6 g Natriumazid wird 2 Tage bei Raumtemperatur weitergerührt. Das Natriumazid wird abgesaugt und die Lösung nach dem Einengen und anschließender Wasserfällung wie üblich aufgearbeitet. Das Rohprodukt wird an 300 g Kieselgel mit einem Methylenchlorid-Aceton-Gradienten (0-30 % Aceton) gereinigt. Ausbeute 3,6 g 21-Acetoxy-11β-hydroxy-17a-methoxymethoxy-D-homo-1,4-pregnadien-3,20-dion. Schmelzpunkt 146 °C.

## Beispiel 2

21-Acetoxy-11β-hydroxy-17a-methoxymethoxy-D-homo-4-pregnen-3,20-dion

1,92 g Tristriphenylphosphinrhodium(I)-chlorid werden in einem Gemisch aus 30 ml Methanol und 90 ml Benzol gelöst und 30 Minuten vorhydriert. Nach Zugabe von 2,4 g 21-Acetoxy-11β-hydroxy-17a-methoxymethoxy-D-homo-1,4-pregnadien-3,20 dion hydriert man 5,5 Stunden unter Normaldruck. Man engt die Lösung am Rotationsverdampfer ein und reinigt den Rückstand an 300 g Kieselgel mit einem Hexan-Essigester-Gradienten (50-70 % Essigester). Ausbeute 1,6 g 21-Acetoxy-11β-hydroxy-17a-methoxymethoxy-D-homo-4-pregnen-3,20-dion. Schmelzpunkt 141 °C.

## Beispiel 3

11β,21-Dihydroxy-17a-methoxymethoxy-D-homo-1,4-pregnadien-3,20-dion

1,2 g 21-Acetoxy-11β-hydroxy-17a-methoxymethoxy-D-homo-1,4-pregnadien-3,20-dion werden mit einer methanolischen $K_2CO_3$-Lösung verseift und wie üblich aufgearbeitet. Ausbeute 920 mg 11β,21-Dihydroxy-17a-methoxymethoxy-D-homo-1,4-pregnadien-3,20-dion. Schmelzpunkt 201 °C.

## Beispiel 4

21-Acetoxy-11β-hydroxy-17a-methylthiomethoxy-D-homo-1,4-pregnadien-3,20-dion

a) 2,7 g 21-Acetoxy-17a-hydroxy-11β-trifluoracetoxy-D-homo-1,4-pregnadien-3,20-dion werden in 27 ml Dimethylsulfoxid, 40 ml Acetanhydrid und 5,4 ml Eisessig 2 Tage bei Raumtemperatur gerührt. Die Reaktionslösung wird auf eine 10 %ige Natriumcarbonatlösung gegeben und der Niederschlag abfiltriert. Man löst den Rückstand in Methylenchlorid und arbeitet nach dem Neutralwaschen wie üblich auf. Nach der Chromatographie an 300 g Kieselgel mit einem Methylenchlorid-Aceton-Gradienten (0-20 % Aceton) isoliert man 1,9 g 21-Acetoxy-17a-methylthiomethoxy-11β-trifluoracetoxy-D-homo-1,4-pregnadien-3,20-dion.

b) 870 mg 21-Acetoxy-17a-methylthiomethoxy-11β-trifluoracetoxy-D-homo-1,4-pregnadien-3,20-dion werden analog Beispiel 1 c) mit Natriumazid/Methanol behandelt. Das Rohprodukt wird an 65 g Kieselgel mit einem Methylenchlorid-Aceton-Gradienten (0-20 % Aceton) gereinigt. Ausbeute 525 mg 21-Acetoxy-11β-hydroxy-17a-methylthiomethoxy-D-homo-1,4-pregnadien-3,20-dion. Schmelzpunkt 148 °C.

## Beispiel 5

21-Acetoxy-9α-fluor-11β-hydroxy-17a-methoxymethoxy-D-homo-1,4-pregnadien-3,20-dion

a) 5,0 g 21-Acetoxy-9α-fluor-11β,17a-dihydroxy-D-homo-1,4-pregnadien-3,20-dion werden analog Beispiel 1 a) mit Trifluoressigsäureanhydrid zu 5,7 g 21-Acetoxy-9α-fluor-17a-hydroxy-11β-trifluoracetoxy-D-homo-1,4-pregnadien-3,20-dion umgesetzt.

b) Unter den Bedingungen des Beispiels 1 b) werden 5,5 g 21-Acetoxy-9α-fluor-17a-hydroxy-11β-trifluoracetoxy-D-homo-1,4-pregnadien-3,20-dion in Methylenchlorid mit Formaldehyddimethylacetal behandelt. Man erhält 3,6 g 21-Acetoxy-9α-fluor-17a-methoxymethoxy-11β-trifluoracetoxy-D-homo-1,4-pregnadien-3,20-dion.

c) Eine Lösung von 3,0 g 21-Acetoxy-9α-fluor-17a-methoxymethoxy-11β-trifluoracetoxy-D-homo-1,4-pregnadien-3,20-dion in 120 ml Methanol wird analog Beispiel 1 c) mit Natriumazid gerührt und aufgearbeitet. Ausbeute 1,4 g 21-Acetoxy-9α-fluor-11β-hydroxy-17a-methoxymethoxy-D-homo-1,4-pregnadien-3,20-dion. Schmelzpunkt 178 °C.

Beispiel 6

21-Butyryloxy-11β-hydroxy-17a-methoxymethoxy-D-homo-1,4-pregnadien-3,20-dion

0,5 g 11β,21-Dihydroxy-17a-methoxymethoxy-D-homo-1,4-pregnadien-3,20-dion werden in 5 ml Pyridin und 2,5 ml Buttersäureanhydrid 2 Stunden bei Raumtemperatur gerührt. Nach der Eiswasserfällung und üblichen Aufarbeitung reinigt man das Rohprodukt an 50 g Kieselgel mit einem Methylenchlorid-Aceton-Gradienten (0-20 % Aceton). Ausbeute 443 mg 21-Butyryloxy-11β-hydroxy-17a-methoxymethoxy-D-homo-1,4-pregnadien-3,20-dion. Schmelzpunkt 124 °C.

Beispiel 7

21-Acetoxy-17a-ethoxymethoxy-11β-hydroxy-D-homo-1,4-pregnadien-3,20-dion

a) 1,4 g 21-Acetoxy-17a-hydroxy-11β-trifluoracetoxy-D-homo-1,4-pregnadien-3,20-dion werden in 10 ml wasserfreiem Methylenchlorid und 7 ml Formaldehyddiethylacetal gelöst und portionsweise mit einem Gemisch aus 2,0 g Kieselgur W 20 und 1,0 g Phosphorpentoxid versetzt. Man rührt 24 Stunden bei Raumtemperatur nach, saugt ab und eluiert den Rückstand nochmals mit Methylenchlorid, das 3 bis 5 % Triethylamin enthält. Das Rohprodukt wird an 150 g Kieselgel mit einem Methylenchlorid-Aceton-Gradienten (0-15 % Aceton) gereinigt. Ausbeute 680 mg 21-Acetoxy-17a-ethoxymethoxy-11β-trifluoracetoxy-D-homo-1,4 pregnadien-3,20-dion.

b) 600 mg 21-Acetoxy-17a-ethoxymethoxy-11β-trifluoracetoxy-D-homo-1,4-pregnadien-3,20-dion werden analog Beispiel 1 c) mit Natriumazid zu 410 mg 21-Acetoxy-17a-ethoxymethoxy-11β-hydroxy-D-homo-1,4-pregnadien-3,20-dion umgesetzt und aufgearbeitet. Schmelzpunkt 112 °C.

Beispiel 8

21-Chlor-17a-ethoxymethoxy-9α-fluor-11β-hydroxy-D-homo-1,4-pregnadien-3,20-dion

a) 4,0 g 21-Chlor-9α-fluor-11β,17a-dihydroxy-D-homo-1,4-pregnadien-3,20-dion werden unter den Bedingungen des Beispiels 1 a) mit Trifluoressigsäureanhydrid zu 3,8 g 21-Chlor-9α-fluor-17a-hydroxy-11β-trifluoracetoxy-D-homo-1,4-pregnadien-3,20-dion umgesetzt.

b) Analog Beispiel 7 a) werden 3,5 g 21-Chlor-9α-fluor-17a-hydroxy-11β-trifluoracetoxy-D-homo-1,4-pregnadien-3,20-dion in Methylenchlorid mit Formaldehyddiethylacetal behandelt. Man isoliert 2,3 g 21-Chlor-17a-ethoxymethoxy-9α-fluor-11β-trifluoracetoxy-D-homo-1,4-pregnadien-3,20-dion.

c) Eine Lösung von 2,0 g 21-Chlor-17a-ethoxymethoxy-9α-fluor-11β-trifluoracetoxy-D-homo-1,4-pregnadien-3,20-dion wird analog Beispiel 1 c) mit Natriumazid gerührt und aufgearbeitet. Ausbeute 820 mg 21-Chlor-17a-ethoxymethoxy-9α-fluor-11β-hydroxy-D-homo-1,4-pregnadien-3,20-dion. Schmelzpunkt 156 °C.

**Patentansprüche**

1. D-Homo-kortikoide der allgemeinen Formel I

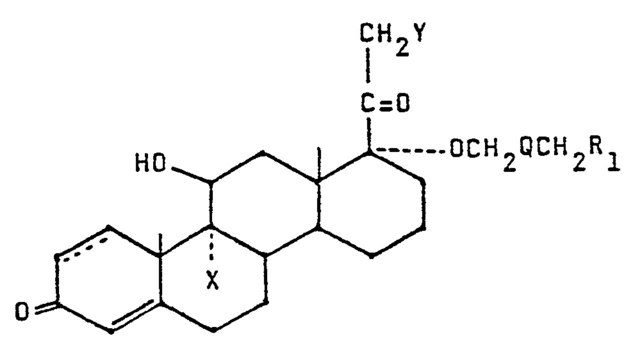

(I)

worin

Die Bindung ⋯ eine Einfachbindung oder eine Doppelbindung

Q ein Sauerstoffatom oder ein Schwefelatom
X ein Wasserstoffatom, ein Fluoratom oder ein Chloratom,
$R_1$ ein Wasserstoffatom oder eine niedere Alkylgruppe und
Y ein Fluoratom, ein Chloratom, eine Hydroxygruppe oder eine Acyloxygruppe bedeuten.

2. D-Homo-kortikoide der allgemeinen Formel I gemäß Anspruch 1, worin Y eine Hydroxygruppe oder eine 1 bis 8 Kohlenstoffatome enthaltende Acyloxygruppe bedeutet.

3. 21-Acetoxy-11β-hydroxy-17aα-methoxymethoxy-D-homo-1,4-pregnadien-3,20-dion.

4. 21-Acetoxy-11β-hydroxy-17aα-methoxymethoxy-D-homo-4-pregnen-3,20-dion

5. 11β,21-Dihydroxy-17aα-methoxymethoxy-D-homo-1,4-pregnadien-3,20-dion

6. 21-Acetoxy-11β-hydroxy-17aα-methylthiomethoxy-D-homo-1,4-pregnadien-3,20-dion.

7. 21-Acetoxy-9α-fluor-11β-hydroxy-17aα-methoxymethoxy-D-homo-1,4-pregnadien-3,20-dion.

8. 21-Butyryloxy-11β-hydroxy-17aα-methoxymethoxy-D-homo-1,4-pregnadien-3,20-dion.

9. 21-Acetoxy-17aα-ethoxymethoxy-11β-hydroxy-D-homo-1,4-pregnadien-3,20-dion.

10. 21-Chlor-17aα-ethoxymethoxy-9α-fluor-11β-hydroxy-D-homo-1,4-pregnadien-3,20-dion.

11. Pharmazeutische Präparate enthaltend ein oder zwei D-homo-kortikoide gemäß Anspruch 1 bis 10.

12. Verfahren zur Herstellung von D-homo-kortikoiden der allgemeinen Formel I gemäß Anspruch 1, dadurch gekennzeichnet, daß man in an sich bekannter Weise ein Kortikoid der allgemeinen Formel II

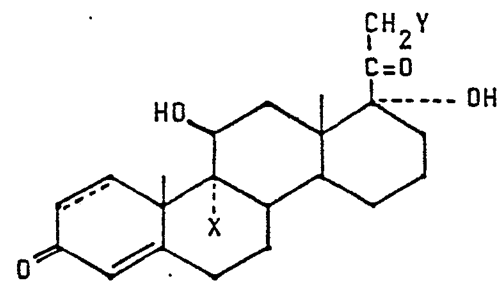

(II)

worin ⋯, X und Y die im Anspruch 1 genannte Bedeutung besitzen, gegebenenfalls nach intermediären Schutz der 11- und 21-Hydroxygruppe mit einem Sulfoxid der allgemeinen Formel III

$$CH_3SOCH_2R_1 \quad (III)$$

oder einem Acetal der allgemeinen Formel IV

$$CH_2(OCH_2R_1)_2 \quad (IV)$$

worin $R_1$ jeweils die im Anspruch 1 genannte Bedeutung besitzt, umsetzt und gegebenenfalls eine vorhandene 21-Estergruppe verseift oder eine 21-Hydroxygruppe verestert oder gegen Halogenatome austauscht.

**Claims**

1. D-homocorticoids of general formula I

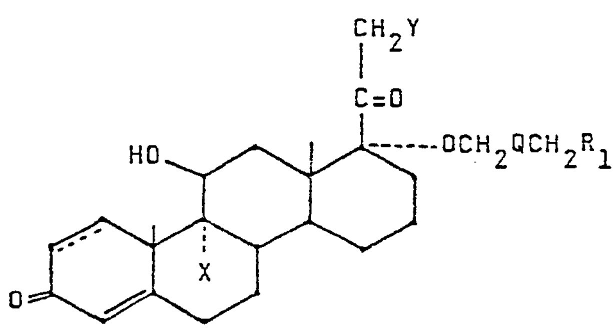

(I)

wherein
the bond ⋯ is a single or double bond,
Q is oxygen or sulphur,
X is hydrogen, fluorine or chlorine,

$R_1$ is hydrogen or a lower alkyl group, and

Y is fluorine, chlorine, hydroxy or an acyloxy group.

2. D-homocorticoids of general formula I according to claim 1 wherein Y is a hydroxy group or an acyloxy group of 1 to 8 carbon atoms.

3. 21-Acetoxy-11β-hydroxy-17aα-methoxymethoxy-D-homo-1,4-pregnadiene-3,20-dione.

4. 21-Acetoxy-11β-hydroxy-17aα-methoxymethoxy-D-homo-4-pregnene-3,20-dione.

5. 11β,21-Dihydroxy-17aα-methoxymethoxy-D-homo-1,4-pregnadiene-3,20-dione.

6. 21-Acetoxy-11β-hydroxy-17aα-methylthiomethoxy-D-homo-1,4-pregnadiene-3,20-dione.

7. 21-Acetoxy-9α-fluoro-11β-hydroxy-17aα-methoxymethoxy-D-homo-1,4-pregnadiene-3,20-dione.

8. 21-Butyryloxy-11β-hydroxy-17aα-methoxymethoxy-D-homo-1,4-pregnadiene-3,20-dione.

9. 21-Acetoxy-17aα-ethoxymethoxy-11β-hydroxy-D-homo-1,4-pregnadiene-3,20-dione.

10. 21-Chloro-17aα-ethoxymethoxy-9β-fluoro-11β-hydroxy-D-homo-1,4-pregnadiene -3,20-dione.

11. Pharmaceutical compositions containing one or two D-homocorticoids according to claims 1 to 10.

12. Process for the preparation of D-homocorticoids of general formula I according to claim 1 characterised in that a corticoid of general formula II

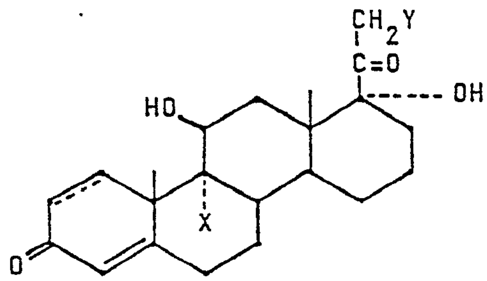

(II)

in which ⎓, X and Y, have the meanings given in claim 1, is reacted in a known manner, optionally after intermediate protection of the 11- and 21-hydroxy groups, with a sulphoxide of general formula III

$$CH_3SOCH_2R_1 \quad (III)$$

or an acetal of general formula IV

$$CH_2(OCH_2R_1)_2 \quad (IV)$$

wherein $R_1$ has the meaning given in claim 1, and if necessary a 21-ester group, when present, is saponified or 21-hydroxy group is esterified or exchanged by a halogen atom.

**Revendications**

1. D-Homo-corticostéroïdes qui répondent à la formule générale I :

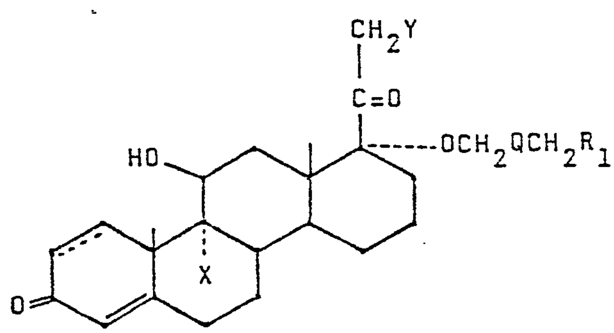

(I)

dans laquelle

la liaison ⎓ représente une liaison simple ou une liaison double,

Q représente un atome d'oxygène ou de soufre,

X représente un atome d'hydrogène, de fluor ou de chlore,

$R_1$ représente un atome d'hydrogène ou un radical alkyle inférieur et

Y représente un atome de fluor ou de chlore ou un radical hydroxy ou acyloxy.

2. D-Homo-corticostéroïdes de formule générale I selon la revendication 1, dans lesquels Y représente un radical hydroxy ou un radical acyloxy contenant de 1 à 8 atomes de carbone.

3. Acétoxy-21 hydroxy-11β méthoxyméthoxy-17aα D-homo-prégnadiène-1,4 dione-3,20.

4. Acétoxy-21 hydroxy-11β méthoxyméthoxy-17aα D-homo-prégnène-4 dione-3,20.

5. Dihydroxy-11β, 21 methoxyméthoxy-17aα D-homo-prégnadiène-1,4 dione-3,20.

6. Acétoxy-21 hydroxy-11β méthylthio-méthoxy-17aα D-homo-prégnadiène-1,4 dione-3,20.

7. Acétoxy-21 fluoro-9α hydroxy-11β méthoxyméthoxy-17aα D-homo-prégnadiène-1,4 dione-3,20.

8. Butyryloxy-21 hydroxy-11β méthoxyméthoxy-17aα D-homo-prégnadiène-1,4 dione-3,20.

9. Acétoxy-21 éthoxyméthoxy-17aα hydroxy-11β D-homo-prégnadiène-1,4 dione-3,20.

10. Chloro-21 éthoxyméthoxy-17aα fluoro-9α hydroxy-11β D-homo-prégnadiène-1,4 dione-3,20.

11. Compositions pharmaceutiques qui contiennent un ou deux D-homo-corticostéroïdes selon l'une quelconque des revendications 1 à 10.

12. Procédé de préparation de D-homo-corticostéroïdes de formule générale I selon la revendication 1, procédé caractérisé en ce que, en opérant de manière connue, on fait réagir un corticostéroïde de formule générale II :

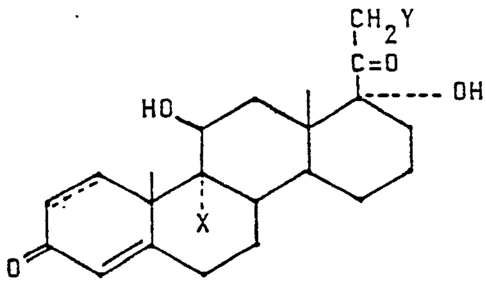

(II)

dont on a éventuellement protégé intermédiairement les radicaux hydroxy en 11 et en 21 (dans cette formule II les symboles ⎓, X et Y ont les significations données à la revendication 1), avec un sulfoxyde de formule générale III :

$$CH_3SOCH_2R_1 \quad \text{(III)}$$

ou avec un acétal de formule générale IV :

$$CH_2(OCH_2R_1)_2 \quad \text{(IV)}$$

(dans chacune de ces formules le symbole $R_1$ a la signification donnée à la revendication 1) et éventuellement on saponifie un radical d'ester en 21 ou on estérifie un radical hydroxy en 21 ou on l'échange contre un atome d'halogène.